# EUROPEAN PATENT SPECIFICATION

(11) **EP 4 395 666 B1**
(45) Date of publication and mention of the grant of the patent: **09.12.2026**
(21) Application number: 22765880.4
(22) Date of filing: 18.08.2022
(51) Int. Cl.: A61B 17/20, A61B 17/34, A61B 17/28, A61B 17/32

(54) **TISSUE EXTENSION**
GEWEBEERWEITERUNG
EXTENSION DE TISSU

(30) Priority: 31.08.2021 GB 202112387
(43) Date of publication of application: 10.07.2024
(73) Proprietor: Compenso Medtech AS, 4319 Sandnes (NO)
(72) Inventor: ENGSETH, Lars, 0778 Oslo (NO); GRØNSUND, Jørgen, 4319 Sandnes (NO); AUNAN, Eirik, 2614 Lillehammer (NO)
(74) Representative: Dehns
(86) International application number: PCT/EP2022/073087
(87) International publication number: WO 2023/030906

(56) References cited:
- EP-B1- 0 951 243
- CN-A- 103 505 266
- US-A1- 2014 039 523
- US-A1- 2015 032 143
- US-A1- 2020 214 766
- US-B1- 6 551 338
- US-B2- 7 740 623
- SIONG FONG TECK ET AL: "Efficacy and Safety of Functional Medial Ligament Balancing With Stepwise Multiple Needle Puncturing in Varus Total Knee Arthroplasty", THE JOURNAL OF ARTHROPLASTY, ELSEVIER, AMSTERDAM, NL, vol. 35, no. 2, 11 September 2019 (2019-09-11), pages 380 - 387, XP086020110, ISSN: 0883-5403, [retrieved on 20190911], DOI: 10.1016/J.ARTH.2019.09.005

## Description

The invention relates to tissue extension techniques, especially ligament extension techniques which may be applied in processes like ligament balancing in total knee arthroplasty.

In total knee arthroplasty (TKA) of the varus knee there is shortening of the medial structures and lengthening of the lateral structures. Varus knee is the most frequent deformity encountered during TKA. Although controversial as a main factor of implant survival, femorotibial alignment remains a major objective in total knee replacement. Bone deformity is addressed by bone cuts perpendicular to the mechanical axis. In the varus knee, bone cuts perpendicular to the mechanical axis will induce a trapezoid-shaped articular femorotibial gap with a short medial side. It is therefore often necessary to balance the soft tissues on the medial side to provide symmetric laxity. This can be achieved by loosening one or more of the medial soft tissues sequentially, until symmetric laxity is achieved.

Ligament balancing is a well-known procedure and is considered a prerequisite for good function and survival in TKA. Ligament balancing can be established through different techniques. The techniques often focus on medial collateral ligament (MCL) lengthening. One conventional technique consists in subperiosteal release at the MCL foot. Another technique, known as "Bellemans' technique" is a well proven technique involving multiple needle punctures using a 19-gauge needle to puncture the medial collateral ligament multiple times in order to allow a progressive increase in laxity and therefore balances the knee. This technique typically involves anywhere from 5-35 punctures with 3-5 mm spacing needed to get the desired laxity of 2-4 mm increased laxity in extension and 2-6 mm in flexion. However, this does not allow precise quantification of the release. The wide range in the number of punctures needed to achieve success in Bellemans' technique leads to a lack of reproducibility and reliability. A typical procedure using Bellemans' technique is to puncture the ligament five times, then retest the knee balancing to see how much extension of the ligament has been achieved. If further extension is required then a further five punctures are made and the knee is tested again. This process continues until sufficient extension of the ligament has been achieved. The number of punctures required for a given extension cannot be predicted reliably in advance.

These issues are not specific to ligament extension in total knee arthroplasty. Similar issues can also arise in other circumstances where tissue extension is sought.

US 2015/0032143 A1 discloses a tissue mesher comprising one or more blocks having a matrix of holes for clamping a plurality of needles and a supporting framework.

US 7,740,623 B2 discloses a device used to inject a substance into the myocardium. The device includes means for ensuring the needles do not penetrate beyond a desired depth into the myocardium.

US 2014/0039523 A1 discloses an apparatus for resurfacing of skin. The apparatus includes one or more needles adapted to remove a small portion of tissue when inserted into and withdrawn from the skin.

EP 0 951 243 B1 discloses an instrument for stimulation of connective tissues of mammals, by which said connective tissue can be perforated to produce a desirable response such as inflammation, cell proliferation, collagen synthesis, or fibrous healing.

CN 103505266 A discloses a puncture needle used for medial collateral ligament releasing in the TKA (total knee arthroplasty). The puncture needle is composed of a needle handle, a needle rod and a needle head.

US 2020/0214766 A1 discloses a method and apparatus for facilitating stretching of biological material by forming a plurality of micro-slits, which can be formed by using a plurality of cutting arrangements.

According to a first aspect, the invention provides a tool for elongating a fibrous length of tissue according to claim 1.

As each group of cutters is arranged across the width of the tool so as to form interleaved areas of cutting and non-cutting, when the tool is used, each group of cutters will cut some fibres of the fibrous length of tissue while leaving other fibres uncut. This means that each group of cutters takes an approach similar to the Bellemans' technique by making a series of punctures in the fibrous tissue. However, each group of cutters is more well-defined, reliable and repeatable than the individual punctures of Bellemans' technique as they have a defined relationship and they have non-cutting widths separating the cutters so that each cutter in the group is guaranteed to cut different fibres of the tissue. In addition, as the second group of cutters is arranged in parallel with first group of cutters (both are arranged across the width of the tool), but overlapping at least one non-cutting width of the first group, the second group of cutters is guaranteed to cut some fibres that are not cut by the first group. The second group of cutters therefore adds to the total number of severed fibres in a controlled and well-defined, repeatable manner. Together the first and second groups of cutters form a well-defined pattern of cuts that will cut a well-defined number of fibres of the fibrous tissue. This results in a well-defined elongation of the fibrous tissue.

When performing ligament balancing (or otherwise extending a length of fibrous tissue), the amount of elongation that is achieved depends on the number of fibres that are cut. The fibrous length of tissue (e.g. ligament) comprises a large number of parallel fibres that share any applied load between them. When a fibre is cut, that fibre no longer contributes to the load bearing and so the load is distributed amongst the uncut fibres. As the fibres are elastic, the uncut fibres elongate under the increased load, thereby achieving the desired lengthening of the fibrous tissue (e.g. ligament). The defined arrangement of cutters results in a defined pattern of cut fibres in the tissue which results in a defined and repeatable elongation of the length of fibrous tissue. This makes it much easier for the practitioner to achieve a desired result. For example in the case of a ligament balancing procedure, a preliminary measurement of the imbalance indicates how much lengthening is desired. By selecting a tool with an appropriate arrangement of cutters, the desired lengthening can be achieved in a single cutting operation (i.e. a single application of the tool) in a predictable and repeatable manner. This achieves all the benefits of Bellemans' technique but with the additional benefit of a fast and accurate procedure. In addition, there is a reduction in the training requirements for applying the technique as consistent results can be achieved without requiring lots of practice and experience.

In some embodiments the tool may further comprising: at least one further group of cutters, the further group of cutters comprising a plurality of cutters spaced apart across the width of the tool so as to form an interleaved set of cutting widths and non-cutting widths; and wherein for each further group of cutters, each cutter of that group is positioned so as to partially overlap at least one non-cutting width of an adjacent group of cutters.

Further groups of cutters increase the number of fibres that are cut in the same way as the second group of cutters increases the number of cut fibres with respect to the first group. Each further group again adds a defined pattern to the overall pattern, each cutting some fibres that are not cut by an adjacent group. Overall, the groups together therefore provide a consistent amount of cutting. The tool may have three, four, five or more groups of cutters, each arranged with partial overlaps so that each additional group adds an additional amount of cutting and thus provides an additional amount of lengthening of the fibrous tissue. In this way, different tools with different numbers of groups, and/or with different amounts of cutting can be formed, each for a different, but well-defined degree of cutting and thus a different, but well-defined degree of lengthening.

In addition to being overlapped with a non-cutting length of at least one adjacent group, in some preferred embodiments each group, or at least as many groups as possible, also have each cutter positioned so as to partially overlap at least one non-cutting width of the first group of cutters. In some embodiments each group, has each cutter positioned so as to partially overlap at least one non-cutting width of each other group.

The cutters of each group of cutters need not necessarily be exactly in line with each other across the width of the tool. However, it is convenient and preferred that each group of cutters comprises a row of cutters. Arranging the cutters in a row means that the individual cutters are all positioned at the same position with respect to the length of the fibrous tissue and will therefore all sever fibres of the fibrous tissue at the same distance along the length of the fibrous tissue. Different groups of cutters can then readily be spaced apart along the length of the tissue to cut fibres at different points along the length.

The groups of cutters are preferably spaced apart along the length of the tool. Each group then cuts a different lengthwise portion of the length of fibrous tissue, thereby spacing out the cuts along the length of the tissue. This can spread out the stress on the remaining fibres along the length of the tissue, reducing the impact of the procedure and facilitating recovery as the individual cuts are spread over a larger area of tissue. The spacing of the groups of cutters need not be regular, i.e. the spacing between adjacent groups may vary along the length of the tool. However, in some preferred embodiments the groups of cutters are regularly spaced so that the cuts (and stress) are evenly spread out along the length of the tool and along the impacted length of fibrous tissue.

Within each group of cutters, the spacing of the cutters need not be regular. Indeed it is not necessary for each cutter to be the same length as the other cutters in the group. Irregular lengths of cutters and spacings (non-cutting widths) can still be used and coordinated with cutters in adjacent groups so as to achieve the desired overlap and cutting quantities. However, in preferred embodiments each group of cutters comprises a regularly spaced set of cutters. In some embodiments, each cutter within a group is the same width as other cutters within that group. Similarly, each non-cutting width within a group may be the same width as other non-cutting widths within that group. Such regular arrangements have the advantage of spreading out the cuts as evenly as possible across the width of the tool (and therefore across the width of the fibrous tissue), thereby spreading the load and trauma across the width of the tissue.

Each group of cutters may have its own distinct arrangement of cutters (cutting widths) and spaces (non-cutting widths). The different groups need not have the same arrangement, while still providing well-defined cutting widths and overlaps between cutting widths and non-cutting widths. However, it is convenient and preferable that each group of cutters has the same arrangement of cutters and wherein each group of cutters is offset, in a width direction, from each adjacent group of cutters by a same offset amount. Having the same arrangement and an offset means that each cutter of each group has the same relationship to a corresponding cutter in the adjacent group, i.e. the offset applies to each and every cutter in the group. This ensures that the new fibres (i.e. those cut by one group that are not cut by the adjacent group) are also evenly spread across the width of the tool and the width of the fibrous tissue, thereby spreading the change in load and the stress across the fibrous tissue. When each group has the same pattern of cutting widths and non-cutting widths and each is progressively offset by the same amount in the width direction, if the groups are also evenly spaced in a length direction then the cutters will form a slanted or tilted two-dimensional array of cutters. When the cutters of each group are regularly spaced in the width direction then the two-dimensional array of cutters will form a slanted or tilted grid of cutters. As noted above, this distributes the stress and trauma as evenly as possible within the tool area.

Each group of cutters may be considered to have a combined cutting width, being a sum of all individual cutting widths in the group; and a combined non-cutting width, being a sum of all individual non-cutting widths in the group. A ratio of combined cutting width to combined non-cutting width is approximately the same for each group. By having the same ratio of cutting width to non-cutting width, the tool can ensure that the same proportion of the width of the fibrous tissue is cut by each row. For example, it may be desired to have each group of cutters cut approximately 10% or 20% or 30% (or indeed any ratio) of the fibres in the length of fibrous tissue. Each group therefore cuts the same proportion of fibres such that the number of groups and the consistent degree of overlap results in a linear progression of fibre-cutting as more groups are added to the tool. The degree of lengthening of certain tissues, e.g. ligaments, and particularly the medial collateral ligament (MCL) is found to be linear with the number of such groups of cutters so that an amount of lengthening can readily be selected simply by selecting the appropriate number of groups (e.g. rows) of cutters in the tool. A single application of the selected tool can then reliably achieve the desired degree of lengthening.

Preferably each group of cutters also has the same combined cutting width. Similarly, each group of cutters preferably has the same combined non-cutting width. That is all groups cut the same amount. Or to put it another way, a combined cutting width of the first group of cutters is the same as a combined cutting width of the second group of cutters; and each further group of cutters has a combined cutting width the same as the combined cutting width of the first group of cutters.

The number of cutters in each group may be selected according to the application. The number of cutters, together with the width of each cutter, determines the total amount of cutting. A larger number of cutters helps to spread the cutting out across the width of the tissue. In some embodiments each group has at least five cutters; preferably at least six cutters; more preferably at least seven cutters. A larger number of cutters also allows the tool to cover a wider range of tissue widths while still providing a reliable cutting ratio for each width of tissue. For example, the width of certain ligaments, such as the MCL can vary from one patient to the next. In humans the MCL can vary typically between about 14 mm and 20 mm in width (although in some cases it may be wider or narrower). Ideally the tool is wide enough to cover the wider target tissues, but has sufficient numbers of cutters across the width that when it cuts a narrower target tissue it still spreads the cutting load across several cutting sites. It may be noted that when the cutters are evenly distributed across the width, the cutting ratio can be the same regardless of the width of the target tissue.

Accordingly, in some embodiments each group of cutters extends across a width of at least 20 mm, optionally at least 30 mm. In some examples each group of cutters extends across a common width portion of the tool, the common width portion having a width of at least 20 mm, optionally at least 30 mm. The common width portion is thus shared and covered by each and every group of cutters, although as discussed above, the cutters may be offset or differently arranged across that common width. The common width may be larger than the target tissue, e.g. at least 20 mm or at least 30 mm (or for other tissues it may of course by significantly larger or smaller).

The cutters of the first group of cutters are overlapped with the cutters of the second group of cutters. The cutters of adjacent groups may be overlapped to varying degrees. In some embodiments the cutters may be overlapped such that cutters from adjacent groups may align with at least some of the same fibres. This may result in the same fibre being cut twice by different groups of cutters. This does not necessarily matter as the fibres will heal in due course in the extended configuration. Additionally, while the cutters of the first group and the cutters of the second group overlap, an individual cutter does not necessarily always cut all fibres across its width; some fibres slide or glide out of the way of the cutter as it is applied so that fibres near the edge of a cutter may not actually be severed by the cutter. Thus some small degree of overlap may not necessarily result in multiple cuts of the same fibre. Nevertheless, in some embodiments, significant overlap of cutters may be used such that multiple cuts of the same fibre will inevitably occur from groups with overlapped cutters. As noted above, this is not necessarily a problem, but what is important is that each group cuts at least some new fibres so that the number of groups defines a consistent and reliable total number (or proportion) of cut fibres, thereby defining a consistent degree of lengthening to be achieved with the tool. In some embodiments the overlap, in a width direction of the tool, between the cutters of one group and any other group is no more than 80%, preferably no more than 70%. In some embodiments the overlap, in a width direction of the tool may be about two thirds.

The width of each cutter may vary according to the specific operation, e.g. according to the tissue to be lengthened and the degree of lengthening desired. In some examples, each cutter has a cutting width of at least 1 mm, preferably at least 1.5 mm. Cutters smaller than about 1 mm have been found not to cut enough to work on certain fibrous tissues, such as certain ligaments. Cutters with a cutting width of around 1.6 mm correspond closely to the cutting width of the cannulas used in certain common application of Bellemans' technique and are therefore particularly preferred in some embodiments, e.g. those for MCL ligament balancing operations. In some embodiments each cutter is a cannula. The cannulas may then correspond exactly to those used in Bellemans' technique, or may at least resemble the same shape and cutting action, even if they have a different width for different cutting amounts or ratios.

The tool may take any of a number of forms. For example the tool may take the form of a single rigid structure with the cutters mounted thereon, optionally as an elongate structure with the cutters formed towards one end thereof. The cutters may then be applied to the length of fibrous tissue simply by pressing the cutters into the tissue. The arrangement of cutters will create a corresponding arrangement of cuts in the tissue and the tool can then be removed. The tool may comprises an applicator and the groups of cutters may be formed on an insert which is mountable to the applicator. Such arrangements are particularly useful as the number of cutters required for different degrees of lengthening varies, e.g. different numbers of groups or different arrangements of cutters within the groups provide different amounts of cutting. Therefore different arrangements of cutters may be formed on different inserts so that the practitioner can select an appropriate insert, mount it on the applicator and then use the applicator to apply the insert to the length of fibrous tissue. The applicator may be the same for all different forms of insert. Different sized applicators may of course be provided if desired for different applications, e.g. for different ligaments or different tissues. However, a single applicator may be appropriate for a large number of different inserts for different operations.

The applicator may be disposable. As the same applicator can be used for different inserts, manufacturing in bulk may be achieved at low cost. However, in some embodiments the applicator may be reusable. Different inserts may be attached and removed so that the applicator can be used again for another procedure with a different insert. Therefore the insert is preferably replaceable and/or interchangeable. The inserts may be reusable, but are preferably disposable after use.

The applicator may be a gripping device arranged, in use, to press the cutters into the fibrous length of tissue. The applicator may be pliers. A gripping device such as pliers is convenient for allowing the operator to apply pressure to the cutters from a remote position, e.g. using a scissor action to press the cutters into the tissue.

The tool may comprise a back plate arranged facing the cutters and movable relative thereto such that the applicator can press the cutters and the back plate towards one another. The back plate may thereby hold the length of fibrous tissue in place such that the cutters can be pressed into it by the applicator (e.g. by action of the handles of the gripping device or pliers).

The tool may comprises a plate, the plate comprising a hole corresponding positionally to each cutter, and wherein the applicator is arranged to apply the cutters towards and optionally into and/or through the holes after passing through the fibrous length of tissue. The plate may act as the back plate as discussed above. The holes allow for continued movement of the cutters through the tissue so that full and reliable cutting can be achieved based on the widths of the cutters and without any dependence on the lengths of cutters, flatness of back plate or resilience of fibres. Different plates may be provided corresponding to different inserts (and thereby corresponding to different arrangements of cutters). The tool may be fitted with a selected insert of cutters as well as a corresponding selected plate with appropriate holes. However, in some preferred embodiments, the plate is part of the applicator and is common to all inserts. The holes must therefore correspond to all possible arrangements of cutters from all possible inserts, i.e. it must be suitable for applications targeting multiple different degrees of lengthening. In some examples the different inserts (for different lengthenings) comprise different numbers of groups (e.g. rows) of cutters and the plate may then have corresponding groups (e.g. rows) of holes corresponding to all possible groups of cutters that may be used. This makes for a single tool usable with multiple different inserts and capable of use for any desired target lengthening. This greatly simplifies the procedure for the practitioner as, once the desired degree of lengthening has been determined, the practitioner simply needs to select the appropriate tool with the appropriate arrangement of cutters corresponding to that degree of lengthening, insert it into the applicator and apply it to the fibrous tissue to effect the cutting.

The tool preferably comprises an alignment device arranged for placement against the fibrous length of tissue and arranged to align the groups of cutters perpendicular to a length direction of the fibrous length of tissue. The alignment device ensures that the cutters of each group are aligned appropriately perpendicular to the lengths of fibre so that the cutters will cut the appropriate number of fibres. The alignment device may also be arranged to ensure that the tool extends across the fibrous length of tissue so as to cover its full width. For example the alignment device may be positioned near the pivot of a pliers-type tool, with the cutters arranged to extend away from the pivot and the alignment device towards an end of the tool.

It will be appreciated that the tool may be designed for a number of different procedures. For example it could be designed for lengthening a carpal ligament or a lateral collateral ligament (LCL), or it may be designed for ligaments in other joints and locations. However, in some embodiments the tool may be a medial collateral ligament lengthening tool.

The cutters (e.g. the insert) may be made from any suitable material, including metal and/or plastic.

The insert and/or the tool may be designed with rounded corners so as to facilitate easy insertion into a joint area without catching on, or damaging, other tissues.

Certain preferred embodiments of the invention will now be described by way of example only, and with reference to the accompanying drawings in which:
Figure 1a shows a view from above of a tool insert according to an embodiment of the invention;
Figure 1b shows how the tool is aligned with a ligament in use;
Figures 2a and 2b show perspective views of the tool insert of Figure 1;
Figure 3a shows a cross-section of a pliers-type applicator tool;
Figure 3b shows a head of a pliers-type applicator tool
Figure 4 shows an alternative forceps-type applicator tool; and
Figure 5 shows an image of a porcine ligament after application of a tool according to the invention.

The following description is of preferred embodiments of the invention

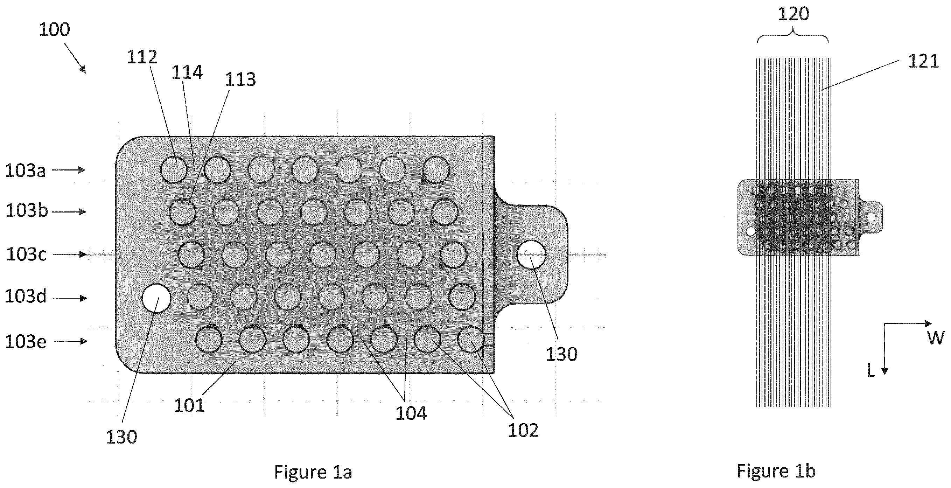

Figure 1a shows a view from above of a tool according to an embodiment of the invention. The tool is for cutting the fibres of a fibrous length of tissue such as a ligament with the aim of causing extension or elongation of that length of tissue. One common application of such elongation is for ligament balancing of the medial collateral ligament (MCL) after total knee arthroplasty operations. The application of this invention is described here in the context of such ligament balancing operations on the MCL, but it will be appreciated that the tool and the technique is applicable in other applications too. For example, the tool may be used in any other application where ligaments are to be elongated such as operations involving ligaments in other joints in the knee (e.g. lateral collateral ligament), hip, elbow, etc. The tool may also be used for lengthening of ligaments such as the carpal ligament. The mechanical principles upon which the tool operates are broadly the same across these various procedures.

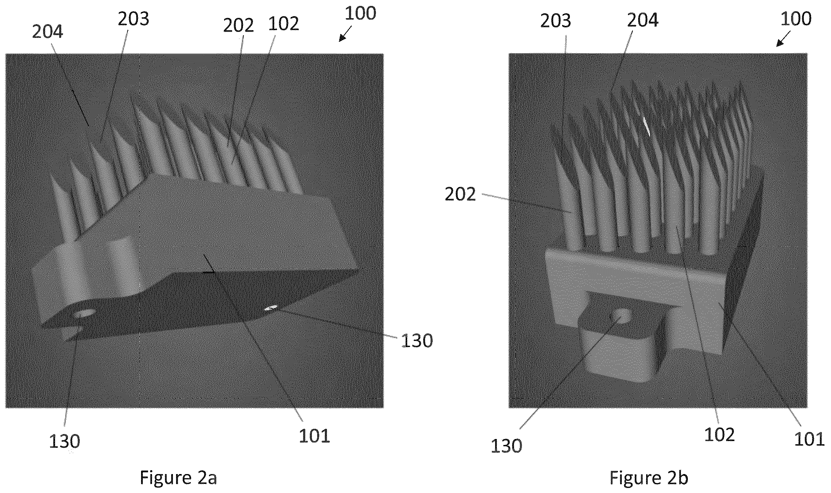

Figure 1 shows a tool 100 designed for use in MCL ligament balancing procedures. The tool 100 is shown from above in Figure 1 and is also shown in two perspective views in Figures 2a and 2b.

The tool 100 is formed from a base 101, with a number of cutters 102 extending from one side of the base 101. The cutters 102 are arranged in a pattern which is made up of five groups 103a-103e of cutters 102. Each group 103a-103e is progressively offset further in a width direction of the tool 100 so that the cutters 102 of each group 103 cut different fibres of the ligament. Together the five groups 103a-e of cutters 102 form a tilted or slanted grid of cutters.

This tool 100 effects the puncturing of Bellemans' technique for ligament balancing by puncturing the tissue in multiple separate places, each puncture (or cut) severing a small number of fibres of the ligament. However, the advantage of this tool over the current application of Bellemans' technique is that the punctures (or cuts) are all made simultaneously and in a pre-arranged pattern so that a predictable and repeatable number of fibres will be cut, thereby achieving a predictable and repeatable extension of the ligament. In addition, the risk of complete detachment (or severing) of the ligament is also reduced, as the tool 100 has a predetermined pattern of cutters 102 which can be designed to ensure that the cuts are spread across the area of the ligament, leaving enough strength left in the ligament.

Each group 103a-e of cutters 102 comprises a set of seven cutters 102. The cutters 102 within each group 103a-e are regularly spaced so that the cutters 102 form cutting widths (i.e. portions of the width of the tool that will cut fibres of the ligament) and the spaces 104 between cutters 102 form non-cutting widths (i.e. portions of the width of the tool that will not cut fibres of the ligament). As the five groups 103a-e are all offset along the width of the tool by different amounts, each group of cutters causes different sets of fibres of the ligament to be cut. Comparing two adjacent groups of cutters, namely groups 103a and 103b, it can be seen that there is an overlap between corresponding cutters in the adjacent groups. For example, cutter 113 of group 103b is partially overlapped with cutter 112 of group 103a as well as being partially overlapped with non-cutting space 114 of group 103a. The part of cutter 113 that overlaps the non-cutting space 114 will cut fibres of the ligament that were not cut by any cutters of group 103a as those fibres passed through the non-cutting space 114. This can be seen from Fig. 1b which shows schematically how the tool is aligned, in use, with the fibres 121 of a ligament 120. The fibres 121 each extend the length of the ligament 120 and the fibres 121 are arranged in parallel across the width of the ligament 120. The tool 100 is arranged so that the tool width is parallel to the width of the ligament 120, i.e. so that each group 103a-e of cutters 102 extends across the width of the ligament 120 such that it will cut some of the fibres 121 but will not cut others of the fibres 121. In Figure 1b, the length direction is indicated by arrow L and the width direction by arrow W.

In Figure 1a, each of the five groups 103a-e is offset to the right, along the width of the tool 100 by a different amount. Each of the second to fifth groups 103b-e is offset by a multiple of a base offset amount. The base offset amount is the offset of the second group 103b relative to the first group 103a. Thus any pair of adjacent groups 103a-e has the same offset between those two groups 103a-e. Each group 103b-e therefore has cutters 102 positioned to overlap a non-cutting space 104 of the group 103a-d above. Thus each additional group 103b-e beyond the first group 103a cuts a new set of fibres that were not cut by the group above. In this one, each additional group adds to the total number of fibres 121 that are cut by the tool 100 as a whole. As more fibres 121 of the ligament 120 are cut, the load on the ligament is shared by fewer fibres and thus it elongates under that load. Thus, but carefully selecting the number of groups 103a-e of cutters 102 in the tool 100, the total amount of cutting, and hence the total amount of lengthening can be selected. For example, a tool 100 with only the first two rows 103a-b of Figure 1a will cut a certain quantity of fibres 121 and will result in a certain degree of lengthening. By comparison, a tool 100 with the first three rows 103a-c of Figure 1a will cut a greater quantity of fibres 121 and will result in a greater degree of lengthening. It has been found that in certain situations, there is a substantially linear relationship between the number of cut fibres 121 and the degree of lengthening of the ligament 120. This is the case with the MCL. Accordingly, different tools 100 can readily be made with different numbers of groups 103a-e of cutters 102, each of those tools 100 corresponding to a different degree of lengthening of the target tissue (e.g. ligament). This greatly simplifies and speed up the procedure as it removes the iterative part of Bellemans' process in which repeated puncturing and re-measuring is required until the desired lengthening is achieved.

Within each group 103a-e of cutters 102, each cutter 102 in the group 103a-e may be identical or they may be of different sizes/widths. However, when the widths of all cutters 102 are added together, they provide a total cutting width for that group 103a-e. In the case where all cutters 102 of a group 103a-e are identical then the total cutting width with simply be the width of one cutter 102 multiplied by the number of cutters 102 in the group 103a-e. A similar calculation may be performed for the total non-cutting width of the group 103a-e. Again, the majority of non-cutting widths may in some embodiments be the same (although they could also be irregular with irregularly spaced cutters 102). However, there are also non-cutting widths at each end of each group 103a-e which will typically vary between groups 103a-e as the offsets are different. If the number of cutters 102 in each group 103a-e is the same and the tool 100 is rectangular (or at least has parallel sides in the cutting region) then the total cutting width plus the total non-cutting width for each group 103a-e will be constant.

In the embodiment shown in Figures 1a, 2a and 2b, the cutters 102 in each group 103a-e are arranged in a straight row, although it will be appreciated that this is not strictly necessary to achieve the desired amounts of cutting, so long as the right number of fibres 121 are cut by the group 103a-e as a whole.

As can best be seen from the perspective views of Figures 2a and 2b, the cutters 102 in this embodiment are cannulas 202. Each cannula 202 is a hollow tube with a tapered tip 203 that tapers to a point 204. The tapered tip 203 is sharp and acts as a cutting blade, slicing through tissue as it is pressed into the tissue parallel to the axis of the tube. Cannulas 202 are used in this embodiment as they are used as the cutting device in the standard application of Bellemans' technique for ligament balancing, thereby making punctures in the tissue in a similar manner to Bellemans' technique and thereby achieving similar results. However, it will be appreciated that other types of cutters may also be used. For example, a pointed scalpel blade may be used instead to make an incision in the tissue in much the same manner.

In the specific embodiment shown in Figures 1, 2a and 2b, the cannulas 202 are 1.6 mm diameter cannulas. The cannulas 202 in each group 103a-e have their centres spaced 3 mm apart. This leaves a 1.4 mm non-cutting width between each adjacent pair of cannulas 202 of one group 103a-e. Each cannula 202 may cut fibres 121 across its whole diameter (e.g. across a width of 1.6 mm for a 1.6 mm diameter cannula), but due to the cylindrical shape of the cannulas 202, they may also push some fibres 121 out of the way, i.e. to the sides, rather than cut them. This can result in an effective cutting width that is slightly smaller than the diameter of the cannula 202. This may be taken into account when designing the tool 100, in particular the amount of overlap between cutters 102 of adjacent groups 103a-e as this will affect how many new fibres 121 are cut with each new group 103a-e as well as how many fibres 121 may be cut twice by overlapped cutters 102.

The diameter of cannula 202 (or width of other cutter 102) can of course be different for different applications and in order to achieve different degrees of cutting and different degrees of overlap.

As noted above, in the tool 100 shown in Figures 1a, 2a and 2b, the groups 103a-e get progressively further offset in the width direction according to their position in the length direction. This forms a slanted grid of cutters 102. In the particular example shown in these figures, the angle of the slant is 11.7 degrees, although it will be appreciated that this is just one example and that many other angles are possible. The angle, together with the cutting widths and the lengthwise spacing between groups 103a-e determines the amount of overlap between cutters and therefore determines the number of new fibres cut by each row. For cannulas in the region of 1.6 mm diameter, with centre spacings of 3 mm (in both width and length directions), an optimal angle for the slant of the grid has been found to be between 5 degrees and 25 degrees for medial collateral ligament balancing procedures. An angle of less than 5 degrees leads to more double-cutting of ligaments with normal sized cannulas and does not result in enough additional fibres 121 being cut with each new group 103a-e. An angle greater than 25 degrees just makes the tool 100 wider without adding much benefit. With common sizes and spacings of cutters, such large angles also tend to increase the overlap of cutters, i.e. the amount of double-cutting. In the example shown in Figures 1a, 2a and 2b, each row 103a-e is offset from the previous row 103a-e by 0.6 mm, i.e. offsets are at 0.6 mm, 1.2 mm, 1.8 mm and 2.4 mm.

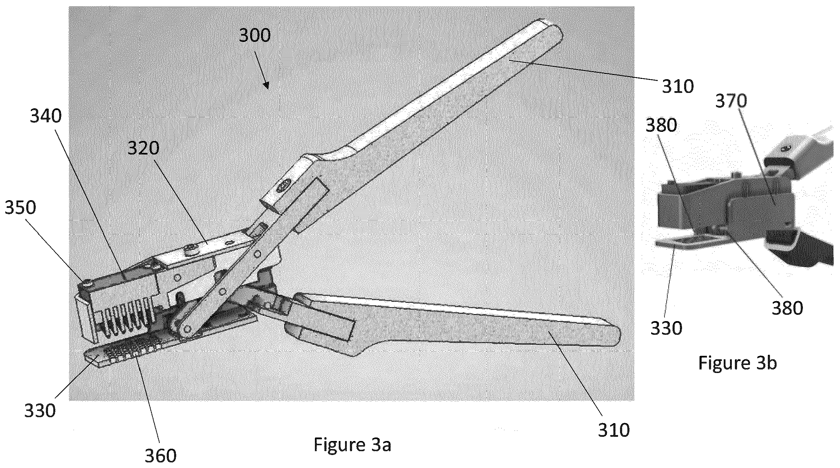

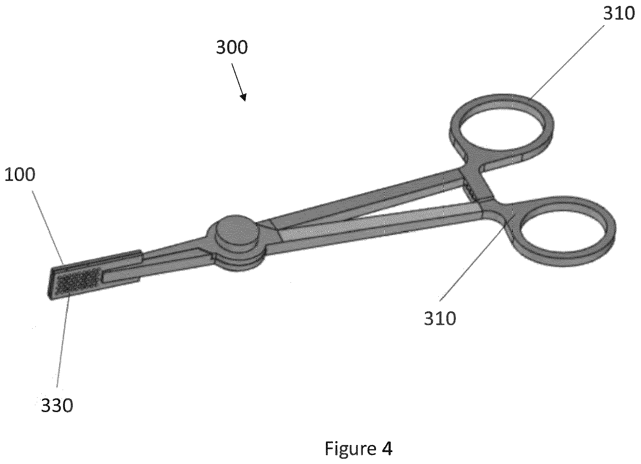

The tool 100 also has two mounting holes 130 that may be used to fix it to an applicator such as those of Figures 3 and 4. In this way the tool 100 becomes a removable insert to a larger tool 300.

Figure 3a shows an applicator 300 in the form of a pliers-type tool. The applicator 300 has handles 310 which, when squeezed together, bring a body 320 and a back plate 330 together. An insert 340 is mounted to the body 320. The insert 340 may be the tool 100 as shown in Figures 1a, 2a and 2b and can be mounted to the body 320 via fixing devices 350 (e.g. screws or clips) passed through the mounting holes 130.

The back plate 330 has holes 360 formed therein, with one hole corresponding to each cutter in the insert 340 and arranged so that the cutters 102 in the insert 340 will pass through the holes 360 upon a full travel of the body 320 when the handles 310 are squeezed fully together. The insert 340 is removable and replaceable and may be disposable so that a new insert can be used for each procedure. The back plate 330 may similarly by removable, replaceable and disposable, but in this embodiment it is a permanent part of the applicator 300. As the back plate 330 is a permanent feature of the applicator 300, it needs to have holes 360 which correspond to any cutters 102 that may be present on any insert 340 that may be used with the applicator 300. As discussed above, different tools 100 (and therefore different inserts 340) may have different numbers of groups 103a-e of cutters 102 depending on the desired degree of lengthening of the ligament that the tool 100 is designed to achieve. As increasing the amount of lengthening simply involves adding additional groups 103a-e of cutters 102, tools 100 for longer elongations can ideally include at least some groups 103a-e of cutters 102 that are also used on tools 100 for shorter elongations. In this way, the back plate 330 can have holes 360 corresponding to each possible group 103a-e of cutters 102 so that the holes 360 are present regardless of whether or not the cutters 102 are present on the particular insert 340.

Figure 3b shows the head of the applicator tool 300, but not in cross-section. This figure shows that side plates 370 of the applicator 300 are formed with an alignment surface 380 parallel with the cutting direction (i.e. the direction in which the cutters 102 are pressed into the ligament) and perpendicular to the back plate 330. The alignment surfaces 380 are, in use, pressed up to the edge of the ligament (or other tissue) so as to align the insert 340 in a defined orientation relative to the fibres. This ensures that the selected pattern of cutters 102 is applied at the correct orientation relative to the fibres 121, thereby ensuring that the correct cutting ratios are achieved and hence that the desired lengthening is achieved reliably.

Figure 4 shows an alternative form of applicator 300, this time in the form of forceps with a back plate 330 and cutting tool 100 provided at one end and operating handles 310 at the other end. The cutting tool 100 may be removable as with the pliers of Figure 3, but may also be a permanent feature of the applicator 300. The applicator 300 may be reusable and a different applicator 300 (with suitably designed tool 100) may be provided for each desired elongation of the target tissue. For example, different applicators 300 may be provided with two rows, three rows, four rows and five rows of cutters, each of these corresponding to different degrees of lengthening of a ligament.

Using the applicator 300 in an MCL ligament balancing procedure may be as follows. First the balance of the knee ligaments is tested as normal to determine the desired degree of lengthening. Next an appropriate insert 340 is selected according to the desired degree of lengthening. The insert 340 may be readily recognised by the number of groups (or rows) 103a-e present on the insert 340. For example, if each row of cutters gives approximately 1 mm of lengthening (purely by way of example) and a 4 mm lengthening is desired, then the insert 340 may have four rows of cutters and is thus easily recognised and verified by eye. The insert 340 is attached to the applicator 300 may fixing devices 350 through mounting holes 130. The applicator 300 is then positioned such that the MCL is between the cutters 102 of the insert 340 and the back plate 330 and such that the edge of the ligament is in contact with both alignment surfaces 380 so that the cutters 102 are appropriately positioned relative to the ligament fibres 121. The handles 310 are squeezed fully together so that the cutters 102 pass through the ligament 120 and through the holes 360 in the back plate 330, thereby cutting fibres 121 of the ligament 120 is a predetermined amount and pattern. The handles 310 are then separated once again and the applicator 300 is removed from the ligament 120. The placement of the punctures in the ligament in the predetermined amount and pattern can be expected to provide the desired degree of lengthening reliably.

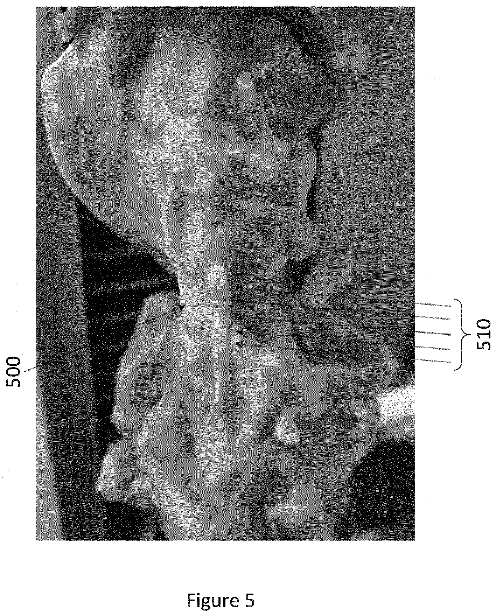

Figure 5 is a photograph of a porcine medial collateral ligament which has been punctured with a tool as discussed above. The holes 500 in the ligament tissue can clearly be seen. Each hole 500 here has been made by a cannula cutter 202 and it can be seen that each row 510 of holes 500 is offset from the other rows 510 such that it cuts different fibres of the ligament (the ligament fibres running approximately perpendicular to the arrows 510 in the figure).

## Claims

1. A tool (100) for elongating a fibrous length of tissue, the tool (100) comprising:
a first group of cutters (103a), the first group of cutters (103a) comprising a plurality of cutters (102, 112, 113) spaced apart across a width of the tool (100) so as to form an interleaved set of cutting widths and non-cutting widths (104, 114);
a second group of cutters (103b), the second group of cutters (103b) comprising a plurality of cutters (102, 112, 113) spaced apart across the width of the tool (100) so as to from an interleaved set of cutting widths and non-cutting widths (104, 114);
wherein the cutters (102, 112, 113) of the first group (103a) of cutters (102, 112, 113) are at least partially overlapped, in a width direction of the tool (100), with the cutters (102, 112, 113) of the second group of cutters (103b); and **characterised in that**:
each cutter (102, 112, 113) of the second group of cutters (103b) is positioned so as to partially overlap, in a width direction of the tool (100), at least one non-cutting width (104, 114) of the first group of cutters (103a).

2. A tool (100) as claimed in claim 1, further comprising:
at least one further group of cutters (103c, 103d, 103e), the further group of cutters (103c, 103d, 103e) comprising a plurality of cutters (102, 112, 113) spaced apart across the width of the tool (100) so as to form an interleaved set of cutting widths and non-cutting widths (104, 114); and
wherein for each further group of cutters (103c, 103d, 103e), each cutter (102, 112, 113) of that group (103c, 103d, 103e) is positioned so as to partially overlap, in a width direction of the tool (100), at least one non-cutting width (104, 114) of an adjacent group of cutters (103a, 103b, 103c, 103d, 103e).

3. A tool (100) as claimed in claim 2, wherein each group of cutters (103a, 103b, 103c, 103d, 103e) has the same arrangement of cutters (102, 112, 113) and wherein each group of cutters (103a, 103b, 103c, 103d, 103e) is offset, in a width direction, from each adjacent group of cutters (103a, 103b, 103c, 103d, 103e) by a same offset amount.

4. A tool (100) as claimed in any preceding claim, wherein each group of cutters (103a, 103b, 103c, 103d, 103e) comprises a row of cutters (102, 112, 113).

5. A tool as claimed in any preceding claim, wherein the groups of cutters (103a, 103b, 103c, 103d, 103e) are spaced apart along the length of the tool (100); and/or
wherein each group of cutters (103a, 103b, 103c, 103d, 103e) comprises a regularly spaced set of cutters (102, 112, 113); and/or
wherein each group (103a, 103b, 103c, 103d, 103e) has at least five cutters (102, 112, 113); preferably at least six cutters (102, 112, 113); more preferably at least seven cutters (102, 112, 113).

6. A tool (100) as claimed in any preceding claim, wherein each group of cutters (103a, 103b, 103c, 103d, 103e) has a combined cutting width, being a sum of all individual cutting widths in the group (103a, 103b, 103c, 103d, 103e); and a combined non-cutting width, being a sum of all individual non-cutting widths (104,114) in the group (103a, 103b, 103c, 103d, 103e); and
wherein a ratio of combined cutting width to combined non-cutting width is approximately the same for each group (103a, 103b, 103c, 103d, 103e).

7. A tool (100) as claimed in any preceding claim, wherein the overlap, in a width direction of the tool (100), between the cutters of one group (103a, 103b, 103c, 103d, 103e) and any other group (103a, 103b, 103c, 103d, 103e) is no more than 80%, preferably no more than 70%.

8. A tool (100) as claimed in any preceding claim, wherein each group (103a, 103b, 103c, 103d, 103e) of cutters (102, 112, 113) extends across a width of at least 20 mm, optionally at least 30 mm; optionally
wherein each group of cutters (103a, 103b, 103c, 103d, 103e) extends across a common width portion of the tool (100), the common width portion having a width of at least 20 mm, optionally at least 30 mm.

9. A tool (100) as claimed in any preceding claim, wherein each cutter (102, 112, 113) has a cutting width of at least 1 mm, preferably at least 1.5 mm; and/or
wherein each cutter (102, 112, 113) is a cannula (202).

10. A tool (100) as claimed in any preceding claim, wherein the tool (100) comprises an applicator (300) and wherein the groups of cutters are formed on an insert (340) which is mountable to the applicator (300); optionally
wherein the insert (340) is replaceable and/or interchangeable.

11. A tool (100) as claimed in claim 10, wherein the applicator (300) is a gripping device arranged, in use, to press the cutters (102, 112, 113) into the fibrous length of tissue; and/or
wherein the applicator (300) is pliers.

12. A tool (100) as claimed in claim 10 or 11, wherein the tool (100) further comprises a plate (330), the plate (330) comprising a hole (360) corresponding positionally to each cutter (102, 112, 113), and wherein the applicator (300) is arranged to apply the cutters (102, 112, 113) towards and optionally into and/or through the holes (360) after passing through the fibrous length of tissue.

13. A tool (100) as claimed in any preceding claim, wherein the tool (100) comprises an alignment device (380) arranged for placement against the fibrous length of tissue and arranged to align the groups of cutters (103a, 103b, 103c, 103d, 103e) perpendicular to a length direction of the fibrous length of tissue.

14. A tool (100) as claimed in any preceding claim, wherein the tool (100) is a medial collateral ligament lengthening tool.

## Patentansprüche

1. Werkzeug (100) zum Verlängern eines faserigen Gewebeabschnitts, wobei das Werkzeug (100) Folgendes umfasst:
eine erste Gruppe von Schneidelementen (103a), wobei die erste Gruppe von Schneidelementen (103a) eine Vielzahl von Schneidelementen (102, 112, 113) umfasst, die über eine Breite des Werkzeugs (100) voneinander beabstandet angeordnet sind, sodass eine abwechselnde Anordnung von Schneidbreiten und Nichtschneidbreiten (104, 114) gebildet wird;
eine zweite Gruppe von Schneidelementen (103b), wobei die zweite Gruppe von Schneidelementen (103b) eine Vielzahl von Schneidelementen (102, 112, 113) umfasst, die über die Breite des Werkzeugs (100) voneinander beabstandet angeordnet sind, sodass eine abwechselnde Anordnung von Schneidbreiten und Nichtschneidbreiten (104, 114) gebildet wird;
wobei sich die Schneidelemente (102, 112, 113) der ersten Gruppe (103a) von Schneidelementen (102, 112, 113) in Breitenrichtung des Werkzeugs (100) zumindest teilweise mit den Schneidelementen (102, 112, 113) der zweiten Gruppe von Schneidelementen (103b) überlappen; und **dadurch gekennzeichnet, dass**:
jedes Schneidelement (102, 112, 113) der zweiten Gruppe von Schneidelementen (103b) so angeordnet ist, dass es in Breitenrichtung des Werkzeugs (100) mindestens eine Nichtschneidbreite (104, 114) der ersten Gruppe von Schneidelementen (103a) teilweise überlappt.

2. Werkzeug (100) nach Anspruch 1, weiter umfassend:
mindestens eine weitere Gruppe von Schneidelementen (103c, 103d, 103e), wobei die weitere Gruppe von Schneidelementen (103c, 103d, 103e) eine Vielzahl von Schneidelementen (102, 112, 113) umfasst, die über die Breite des Werkzeugs (100) voneinander beabstandet angeordnet sind, sodass eine abwechselnde Anordnung von Schneidbreiten und Nichtschneidbreiten (104, 114) gebildet wird; und
wobei für jede weitere Gruppe von Schneidelementen (103c, 103d, 103e) jedes Schneidelement (102, 112, 113) dieser Gruppe (103c, 103d, 103e) so angeordnet ist, dass es in Breitenrichtung des Werkzeugs (100) mindestens eine Nichtschneidbreite (104, 114) einer benachbarten Gruppe von Schneidelementen (103a, 103b, 103c, 103d, 103e) teilweise überlappt.

3. Werkzeug (100) nach Anspruch 2, wobei jede Gruppe von Schneidelementen (103a, 103b, 103c, 103d, 103e) die gleiche Anordnung von Schneidelementen (102, 112, 113) aufweist und wobei jede Gruppe von Schneidelementen (103a, 103b, 103c, 103d, 103e) in Breitenrichtung gegenüber jeder benachbarten Gruppe von Schneidelementen (103a, 103b, 103c, 103d, 103e) um denselben Versatzbetrag versetzt ist.

4. Werkzeug (100) nach einem der vorhergehenden Ansprüche, wobei jede Gruppe von Schneidelementen (103a, 103b, 103c, 103d, 103e) eine Reihe von Schneidelementen (102, 112, 113) umfasst.

5. Werkzeug nach einem der vorhergehenden Ansprüche, wobei die Gruppen von Schneidelementen (103a, 103b, 103c, 103d, 103e) entlang der Länge des Werkzeugs (100) voneinander beabstandet angeordnet sind; und/oder
wobei jede Gruppe von Schneidelementen (103a, 103b, 103c, 103d, 103e) eine regelmäßig beabstandete Anordnung von Schneidelementen (102, 112, 113) umfasst; und/oder
wobei jede Gruppe (103a, 103b, 103c, 103d, 103e) mindestens fünf Schneidelemente (102, 112, 113) aufweist; vorzugsweise mindestens sechs Schneidelemente (102, 112, 113); besonders bevorzugt mindestens sieben Schneidelemente (102, 112, 113).

6. Werkzeug (100) nach einem der vorhergehenden Ansprüche, wobei jede Gruppe von Schneidelementen (103a, 103b, 103c, 103d, 103e) eine kombinierte Schneidbreite aufweist, die der Summe aller einzelnen Schneidbreiten in der Gruppe (103a, 103b, 103c, 103d, 103e) entspricht; und eine kombinierte Nichtschneidbreite, die der Summe aller einzelnen Nichtschneidbreiten (104, 114) in der Gruppe (103a, 103b, 103c, 103d, 103e) entspricht; und
wobei das Verhältnis der kombinierten Schneidbreite zur kombinierten Nichtschneidbreite für jede Gruppe (103a, 103b, 103c, 103d, 103e) annähernd gleich ist.

7. Werkzeug (100) nach einem der vorhergehenden Ansprüche, wobei die Überlappung in Breitenrichtung des Werkzeugs (100) zwischen den Schneidelementen einer Gruppe (103a, 103b, 103c, 103d, 103e) und den Schneidelementen jeder anderen Gruppe (103a, 103b, 103c, 103d, 103e) höchstens 80 %, vorzugsweise höchstens 70 %, beträgt.

8. Werkzeug (100) nach einem der vorhergehenden Ansprüche, wobei sich jede Gruppe (103a, 103b, 103c, 103d, 103e) von Schneidelementen (102, 112, 113) über eine Breite von mindestens 20 mm, optional mindestens 30 mm, erstreckt; optional
wobei sich jede Gruppe von Schneidelementen (103a, 103b, 103c, 103d, 103e) über einen gemeinsamen Breitenabschnitt des Werkzeugs (100) erstreckt, wobei der gemeinsame Breitenabschnitt eine Breite von mindestens 20 mm, optional mindestens 30 mm, aufweist.

9. Werkzeug (100) nach einem der vorhergehenden Ansprüche, wobei jedes Schneidelement (102, 112, 113) eine Schneidbreite von mindestens 1 mm, vorzugsweise mindestens 1,5 mm, aufweist; und/oder
wobei jedes Schneidelement (102, 112, 113) eine Kanüle (202) ist.

10. Werkzeug (100) nach einem der vorhergehenden Ansprüche, wobei das Werkzeug (100) einen Applikator (300) umfasst und wobei die Gruppen von Schneidelementen auf einem Einsatz (340) ausgebildet sind, der an dem Applikator (300) angebracht werden kann; optional
wobei der Einsatz (340) ersetzbar und/oder austauschbar ist.

11. Werkzeug (100) nach Anspruch 10, wobei der Applikator (300) eine Greifvorrichtung ist, die dazu angeordnet ist, bei Gebrauch die Schneidelemente (102, 112, 113) in den faserigen Gewebeabschnitt zu drücken; und/oder
wobei der Applikator (300) eine Zange ist.

12. Werkzeug (100) nach Anspruch 10 oder 11, wobei das Werkzeug (100) weiter eine Platte (330) umfasst, wobei die Platte (330) für jedes Schneidelement (102, 112, 113) ein entsprechend positioniertes Loch (360) umfasst und wobei der Applikator (300) dazu angeordnet ist, die Schneidelemente (102, 112, 113) nach dem Durchdringen des faserigen Gewebeabschnitts auf die Löcher zuzubewegen und sie optional in die Löcher (360) und/oder durch diese hindurchzuführen.

13. Werkzeug (100) nach einem der vorhergehenden Ansprüche, wobei das Werkzeug (100) eine Ausrichtvorrichtung (380) umfasst, die dazu angeordnet ist, an den faserigen Gewebeabschnitt angelegt zu werden, und die dazu angeordnet ist, die Gruppen von Schneidelementen (103a, 103b, 103c, 103d, 103e) senkrecht zu einer Längsrichtung des faserigen Gewebeabschnitts auszurichten.

14. Werkzeug (100) nach einem der vorhergehenden Ansprüche, wobei das Werkzeug (100) ein Werkzeug zur Verlängerung eines medialen Kollateralbandes ist.

## Revendications

1. Outil (100) destiné à allonger une longueur fibreuse de tissu, l'outil (100) comprenant :
un premier groupe d'éléments de coupe (103a), le premier groupe d'éléments de coupe (103a) comprenant une pluralité d'éléments de coupe (102, 112, 113) espacés sur une largeur de l'outil (100) de manière à former un ensemble entrelacé de largeurs coupantes et de largeurs non coupantes (104, 114) ;
un deuxième groupe d'éléments de coupe (103b), le deuxième groupe d'éléments de coupe (103b) comprenant une pluralité d'éléments de coupe (102, 112, 113) espacés sur la largeur de l'outil (100) de manière à former un ensemble entrelacé de largeurs coupantes et de largeurs non coupantes (104, 114) ;
dans lequel les éléments de coupe (102, 112, 113) du premier groupe (103a) d'éléments de coupe (102, 112, 113) chevauchent au moins partiellement, dans le sens de la largeur de l'outil (100), les éléments de coupe (102, 112, 113) du deuxième groupe d'éléments de coupe (103b) ; et **caractérisé en ce que** :
chaque élément de coupe (102, 112, 113) du deuxième groupe d'éléments de coupe (103b) est positionné de manière à chevaucher partiellement, dans le sens de la largeur de l'outil (100), au moins une largeur non coupante (104, 114) du premier groupe d'éléments de coupe (103a).

2. Outil (100) selon la revendication 1, comprenant en outre :
au moins un groupe supplémentaire d'éléments de coupe (103c, 103d, 103e), le groupe supplémentaire d'éléments de coupe (103c, 103d, 103e) comprenant une pluralité d'éléments de coupe (102, 112, 113) espacés sur la largeur de l'outil (100) de manière à former un ensemble entrelacé de largeurs coupantes et de largeurs non coupantes (104, 114) ; et
dans lequel, pour chaque groupe supplémentaire d'éléments de coupe (103c, 103d, 103e), chaque élément de coupe (102, 112, 113) de ce groupe (103c, 103d, 103e) est positionné de manière à chevaucher partiellement, dans le sens de la largeur de l'outil (100), au moins une largeur non coupante (104, 114) d'un groupe adjacent d'éléments de coupe (103a, 103b, 103c, 103d, 103e).

3. Outil (100) selon la revendication 2, dans lequel chaque groupe d'éléments de coupe (103a, 103b, 103c, 103d, 103e) présente le même agencement d'éléments de coupe (102, 112, 113) et dans lequel chaque groupe d'éléments de coupe (103a, 103b, 103c, 103d, 103e) est décalé, dans le sens de la largeur, par rapport à chaque groupe adjacent d'éléments de coupe (103a, 103b, 103c, 103d, 103e) d'une même valeur de décalage.

4. Outil (100) selon une quelconque revendication précédente, dans lequel chaque groupe d'éléments de coupe (103a, 103b, 103c, 103d, 103e) comprend une rangée d'éléments de coupe (102, 112, 113).

5. Outil selon une quelconque revendication précédente, dans lequel les groupes d'éléments de coupe (103a, 103b, 103c, 103d, 103e) sont espacés sur la longueur de l'outil (100) ; et/ou
dans lequel chaque groupe d'éléments de coupe (103a, 103b, 103c, 103d, 103e) comprend un ensemble d'éléments de coupe (102, 112, 113) espacés de façon régulière ; et/ou
dans lequel chaque groupe (103a, 103b, 103c, 103d, 103e) présente au moins cinq éléments de coupe (102, 112, 113) ; préférablement au moins six éléments de coupe (102, 112, 113) ; plus préférablement au moins sept éléments de coupe (102, 112, 113).

6. Outil (100) selon une quelconque revendication précédente, dans lequel chaque groupe d'éléments de coupe (103a, 103b, 103c, 103d, 103e) présente une largeur coupante combinée, qui est une somme de toutes les largeurs coupantes individuelles du groupe (103a, 103b, 103c, 103d, 103e) ; et une largeur non coupante combinée, qui est une somme de toutes les largeurs non coupantes individuelles (104, 114) du groupe (103a, 103b, 103c, 103d, 103e) ; et
dans lequel un rapport de largeur coupante combinée sur largeur non coupante combinée est approximativement le même pour chaque groupe (103a, 103b, 103c, 103d, 103e).

7. Outil (100) selon une quelconque revendication précédente, dans lequel le chevauchement, dans le sens de la largeur de l'outil (100), entre les éléments de coupe d'un groupe (103a, 103b, 103c, 103d, 103e) et tout autre groupe (103a, 103b, 103c, 103d, 103e) n'est pas supérieur à 80 %, préférablement pas supérieur à 70 %.

8. Outil (100) selon une quelconque revendication précédente, dans lequel chaque groupe (103a, 103b, 103c, 103d, 103e) d'éléments de coupe (102, 112, 113) s'étend sur une largeur d'au moins 20 mm, facultativement d'au moins 30 mm ; facultativement
dans lequel chaque groupe d'éléments de coupe (103a, 103b, 103c, 103d, 103e) s'étend sur une partie de largeur commune de l'outil (100), la partie de largeur commune présentant une largeur d'au moins 20 mm, facultativement d'au moins 30 mm.

9. Outil (100) selon une quelconque revendication précédente, dans lequel chaque élément de coupe (102, 112, 113) présente une largeur coupante d'au moins 1 mm, préférablement d'au moins 1,5 mm ; et/ou
dans lequel chaque élément de coupe (102, 112, 113) est une canule (202).

10. Outil (100) selon une quelconque revendication précédente, dans lequel l'outil (100) comprend un applicateur (300) et dans lequel les groupes d'éléments de coupe sont formés sur un insert (340) qui peut être monté sur l'applicateur (300) ; facultativement
dans lequel l'insert (340) est remplaçable et/ou interchangeable.

11. Outil (100) selon la revendication 10, dans lequel l'applicateur (300) est un dispositif de préhension conçu, en utilisation, pour presser les éléments de coupe (102, 112, 113) dans la longueur fibreuse de tissu ; et/ou
dans lequel l'applicateur (300) est une pince.

12. Outil (100) selon la revendication 10 ou la revendication 11, dans lequel l'outil (100) comprend en outre une plaque (330), la plaque (330) comprenant un trou (360) dont la position correspond à chaque élément de coupe (102, 112, 113), et dans lequel l'applicateur (300) est conçu pour appliquer les éléments de coupe (102, 112, 113) vers et facultativement dans et/ou à travers les trous (360) après passage à travers la longueur fibreuse de tissu.

13. Outil (100) selon une quelconque revendication précédente, dans lequel l'outil (100) comprend un dispositif d'alignement (380) conçu pour être placé contre la longueur fibreuse de tissu et conçu pour aligner les groupes d'éléments de coupe (103a, 103b, 103c, 103d, 103e) perpendiculairement à un sens de longueur de la longueur fibreuse de tissu.

14. Outil (100) selon une quelconque revendication précédente, dans lequel l'outil (100) est un outil d'allongement du ligament collatéral médial.
